# EUROPEAN PATENT SPECIFICATION

(11) **EP 0 960 624 B1**
(45) Date of publication and mention of the grant of the patent: **08.10.2003**
(21) Application number: 99106615.0
(22) Date of filing: 31.03.1999
(51) Int. Cl.: A61M 1/16

(54) **Haemodialysis machine**
Hämodialysegerät
Appareil d'hémodialyse

(30) Priority: 01.04.1998 IT BO980210
(43) Date of publication of application: 01.12.1999
(73) Proprietor: BELLCO S.p.A., 20121 Milano (IT)
(72) Inventor: Cianciavicchia, Domenico, 64040 Cavuccio (IT)
(74) Representative: Cerbaro, Elena, Dr.

(56) References cited:
- EP-A- 0 791 368
- US-A- 3 809 243
- US-A- 4 181 610
- US-A- 4 702 829
- US-A- 5 660 722

## Description

The present invention relates to a haemodialysis machine.

As is known, some machines for treating chronic renal failure, generally known as "haemodialysis machines", provide for the patient's blood, after it has been conveyed through a blood purification unit and before being transfused to the patient, to be mixed with a reinfusion liquid so as to reintegrate the solutes dissolved in the blood and to achieve a suitable hydric balance.

In machines of this sort the blood purification unit normally comprises a capillary fibre filter, inside which the patient's blood is put into contact, by the interposition of a semipermeable membrane, with a dialysis liquid, so that the toxic substances present in the blood may pass by osmosis from the patient's blood to the dialysis liquid; a vessel for collecting the dialysis liquid; pumping means for conveying the dialysis liquid from the collection vessel to the capillary fibre filter, and from the capillary fibre filter to a discharge pipe; and finally a first filtering member capable of filtering the dialysis liquid before it reaches the capillary fibre filter.

Haemodialysis machines also comprise pumping means capable of collecting the patient's blood, conveying it through the capillary fibre filter, and finally returning it to the patient's circulation; and a dripping device provided downstream of the capillary fibre filter in order to remove from the purified blood any air bubbles that might cause embolisms or other very serious disorders in the patient.

At present, the reinfusion liquid is mixed with the patient's blood in conjunction with the dripping device, and for this purpose haemodialysis machines comprise a vessel for collecting the reinfusion liquid, and pumping means for supplying the reinfusion liquid from the collection vessel to the dripping device.

In view of the affinity between the reinfusion liquid and the dialysis liquid, some haemodialysis machines use the dialysis liquid itself as reinfusion liquid; however, these do not have the vessel for collecting the reinfusion liquid, and the means for pumping the reinfusion liquid are inserted into the hydraulic circuit that supplies the dialysis liquid to the capillary fibre filter, so as to supply the dialysis liquid also to the dripping device.

In this case, haemodialysis machines also comprise a second filtering member capable of filtering the reinfusion liquid before it reaches the dripping device, so as to prevent the patient from coming into contact with toxic substances suspended in the reinfusion liquid.

The means for pumping the reinfusion liquid are normally inserted into the hydraulic circuit that supplies the dialysis liquid to the capillary fibre filter, downstream of the first filtering member, so that the dialysis liquid, before reaching the dripping device, may be subjected to filtering action by either the first or the second filtering member.

As is known, at the end of treatment for each patient, it is essential to sterilise the haemodialysis machine, in order to avoid any toxic substances or bacteria contained in the blood of the patient that has just been treated from being transferred to the blood of the patient that is to be treated. Methods for sterilising the machine vary according to how many and which components have to be reused.

Unfortunately, haemodialysis machines as described above have the great drawback of requiring particularly careful sterilisation, which puts them out of action for long intervals of time.

In fact, in the machine described above, the second filtering member, because it is relatively expensive, is reused for a certain number of dialysis treatments before being replaced, so as to reduce the costs of the exercise. This need means it is essential to subject the second filtering member to careful sterilisation at the end of each dialysis treatment, necessarily followed by a structural check to assess the filtering capacity.

Sterilisation requires, first, the washing of the second filtering member with a bactericidal liquid, and then the rinsing of the filtering member with uncontaminated water, so as to remove any residual bactericidal liquid. Since the bactericidal liquid is, however, extremely toxic, the rinsing phase has to last for a long time, during which samples of the washing water must be collected periodically for laboratory analysis to check the percentage of bactericidal liquid present in said water, so that the rinsing phase is interrupted only when the residual bactericidal liquid is below a certain threshold of tolerance.

Like the sterilisation, the structural check also requires a long period of time and even then does not guarantee that the second filtering member, despite having successfully passed the structural check, might then rupture during treatment, making it possible for toxic substances to pass into the patient's blood.

A haemodialysis machine which partially overcomes the aforementioned drawbacks is disclosed in US 5,660,722.

US 5,660,722 discloses a haemodialysis machine comprising a purification unit for purifying the blood of the patient from the toxic substances in solution, a liquid reintegrating device for mixing into the blood conveyed through the purification unit a reinfusion liquid for restoring the correct percentage of salts and liquids in the blood, a blood supply circuit for conveying the blood through the purification unit and the liquid reintegrating device, and a liquid supply circuit for conveying a dialysis liquid through the purification unit and the liquid reintegrating device.

The liquid supply circuit is provided with one supplying pipe extending both through the purification unit and the liquid reintegrating device and with one supplying pump located between the purification unit and the liquid reintegrating device for conveying the dialysis liquid along the supplying pipe.

The object of the present invention is to improve the known haemodialysis machines of the above mentioned type.

According to the present invention, there is provided a haemodialysis machine as recited in Claim 1.

The present invention will now be described with reference to the attached drawing, which illustrate a non-exhaustive embodiment thereof, in which:
Figure 1 illustrates diagrammatically, and with parts removed for clarity, a haemodialysis machine produced according to the dictates of the present invention;
Figure 2 illustrates diagrammatically, and with parts removed for clarity, a variant of the haemodialysis machine illustrated in Figure 1.

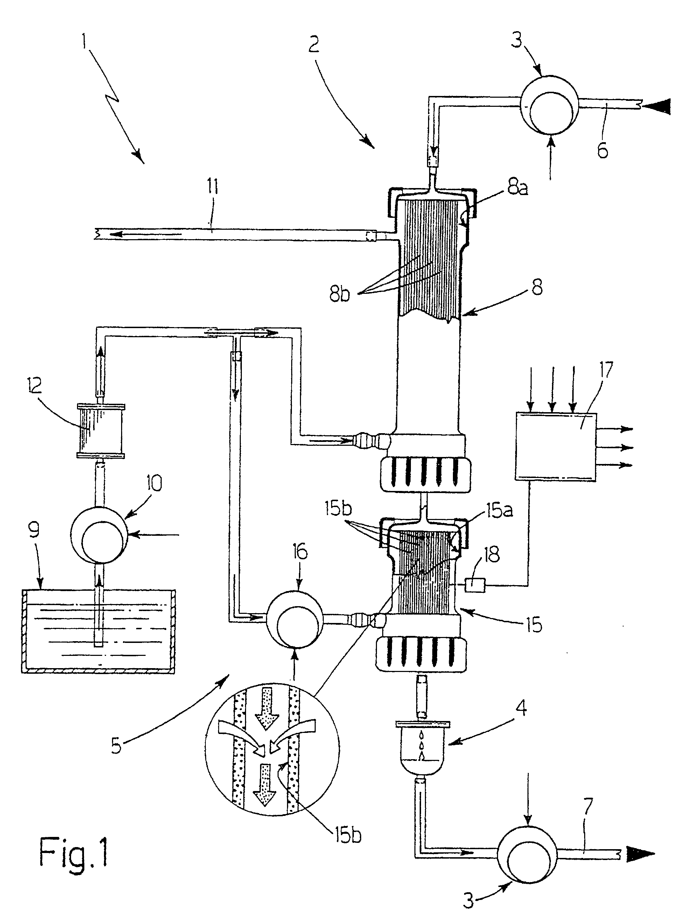

With reference to Figure 1, the reference number 1 indicates, as a whole, a machine for treating patients suffering from chronic renal failure, commonly known as haemodialysis machines.

The haemodialysis machine 1 comprises a blood purification unit 2, through which is conveyed the blood of the patient that is to be purified of any toxic substances; a blood supply circuit, connecting the purification unit 2 to the patient; one or more circulation pumps 3 capable of maintaining the flow of blood that passes through said purification unit 2 at a fixed value; and a dripping device 4 of a known type, which is capable of removing from the blood, when it leaves the purification unit 2, any air bubbles which, if they reached the patient, could cause an embolism or other serious disorders.

The haemodialysis machine 1 also comprises a liquid reintegrating device 5 capable of mixing with the blood leaving the purification unit 2 a saline solution, commonly known as "reinfusion liquid", which can restore the correct proportion of salts in solution in the blood, also reintegrating part of the liquid removed from the blood inside the purification unit 2.

The blood supply circuit comprises two pipes 6 and 7 made of plastic material, the first of which is capable of conveying to the purification unit 2 the blood flowing inside one of the patient's arteries; the second of which is capable of conveying into one of the patient's veins the blood that has just been purified inside said purification unit 2.

In the example illustrated, along each of the two pipes 6 and 7 there is arranged respectively a circulation pump 3 preferably, but not necessarily, of peristaltic type.

With reference to Figure 1, the purification unit 2 comprises a capillary fibre filter 8 of a known type, inside which the patient's blood is put into contact, by the interposition of a semipermeable membrane, with a dialysis liquid so that any toxic substances present in the blood may pass by osmosis from the patient's blood to the dialysis liquid; a vessel 9 for collecting the dialysis liquid; and a pumping unit 10 capable of transferring the dialysis liquid from the vessel 9 to the capillary fibre filter 8. The purification unit 2 also comprises a discharge pipe 11, through which the dialysis liquid contaminated by the toxic solutions transferred from the blood is discharged from the capillary fibre filter 8, and a filtering member 12 capable of filtering the dialysis liquid leaving the vessel 9 in order to keep upstream of the capillary fibre filter 8 any impurities that are present in suspension.

In the example illustrated, the capillary fibre filter 8 is constituted by a liquid-tight container 8a having inside it a bundle of capillary tubes 8b made of semipermeable material. The container 8a is connected to the discharge pipe 11 and to the pumping unit 10 so as to be kept constantly full of dialysis liquid; while the bundle of capillary tubes 8b connects together the pipes 6 and 7 so that the patient's blood, passing through the capillary tubes 8b, can transfer toxic substances in suspension by osmosis to the dialysis liquid that washes the outer surface of said capillary tubes 8b.

With reference to Figure 1, the liquid reintegrating device 5 comprises a mixing and filtering assembly 15 interposed between the blood purification unit 2 and the dripping device 4; a collection vessel for the reinfusion liquid corresponding, in the example illustrated, to the dialysis liquid collection vessel 9; and a circulation pump 16 preferably, but not necessarily, of peristaltic type, capable of conveying to the mixing and filtering assembly 15 part of the dialysis liquid leaving the filtering member 12.

The mixing and filtering assembly 15 is constituted by a capillary fibre filter comprising a liquid-tight container 15a, and a bundle of capillary tubes 15b made of semipermeable material enclosed within said liquid-tight container 15a. The bundle of capillary tubes 15b has passing through it the blood leaving the purification unit 2, or leaving the capillary fibre filter 8, while the container 15a is connected to the circulation pump 16 which is intended to convey into said container 15a reinfusion liquid or dialysis liquid, at a pressure determined so as to cause the reinfusion liquid to pass inside the capillary tubes 15b where said reinfusion liquid is mixed with the blood flowing along said capillary tubes 15b.

The walls of the capillary tubes 15b are made so as to allow the passage of the reinfusion liquid but not of any impurities present in suspension; consequently, the reinfusion liquid, or the dialysis liquid, passing inside the capillary tubes 15b so as to be mixed with the patient's blood, is filtered and leaves all its impurities inside the container 15a and outside the capillary tubes 15b.

In the example illustrated, the capillary fibre filter 8 and the mixing and filtering assembly 15 are of the use-and-throw type and are arranged in tandem with one another, and can if appropriate be incorporated into a single member. The pipe 7 is therefore connected to the outlet of the mixing and filtering assembly 15.

The liquid reintegrating device 5 also comprises means for sensing the presence of the patient's blood inside the container 15a; the presence of blood inside the container 15a being indicative of the fact that one or more capillary tubes 15b are ruptured, and therefore that the mixing and filtering assembly 15 is malfunctioning.

The means for sensing the presence of blood are connected to the central control unit 17, which is capable of controlling the circulation pump 3, the pumping unit 10 and the circulation pump 16, and in general to monitor the functioning of the various components of the machine 1 so as to indicate any malfunctions that would be dangerous for the patient's safety.

Obviously, if conditions that are particularly dangerous for the patient's well-being are ascertained, the control unit 17 can deactivate the machine 1.

In the example illustrated, the sensing means are constituted by an optical sensor 18, which is arranged outside the container 15a, facing a transparent portion of the wall of said container 15a. The optical sensor 18 is capable of sensing a variation in the colour of the reinfusion liquid arising from the presence of blood, so as to notify the control unit 17 of the machine 1, in real time, of the breakage or fissuring of one or more capillary tubes 15b, or a loss of efficiency in the mixing and filtering assembly 15.

According to a variant not illustrated, the liquid reintegrating device 5 is capable of mixing the reinfusion liquid with the patient's blood entering from the purification unit 2. In this case, the mixing and filtering assembly 15 is arranged upstream of the blood purification unit 2, or on the opposite side from the dripping device 4.

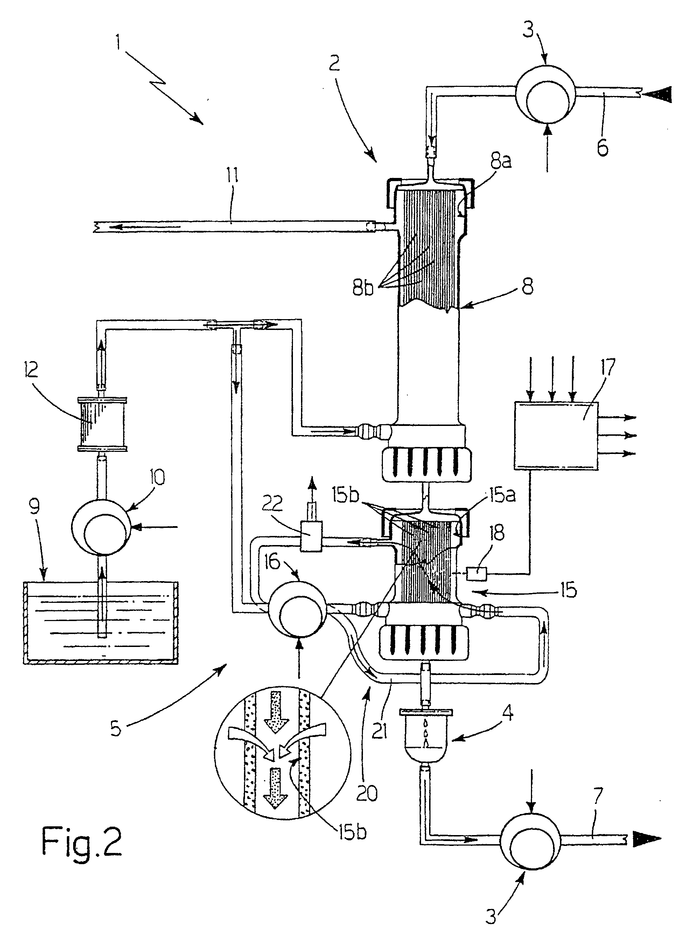

According to the variant illustrated in Figure 2, the machine 1 is provided with a recirculation circuit 20 capable of collecting the reinfusion liquid from one zone of the container 15a and returning it to another part of the same container 15a, so as to create a current capable of preventing any impurities left by the reinfusion liquid outside the capillary tubes 15b from being deposited on the walls of said capillary tubes 15b.

With reference to Figure 2, in the example illustrated the recirculation circuit 20 comprises a pipe 21 made of plastic material connecting together the upper and lower ends of the container 15a of the mixing and filtering assembly 15, and a bleed valve 22 arranged along the pipe 21. Said bleed valve 22 is capable of connecting said pipe 21 to the outside, when commanded, so as to discharge the reinfusion liquid from the container 15a.

The flow of reinfusion liquid inside the container 15a is ensured by the circulation pump 16, which acts simultaneously both on the pipe 21 and on the pipe connecting the mixing and filtering assembly 15 with the vessel 9 downstream of the filtering member 12.

In this case the optical sensor 18 can be arranged along a transparent portion of the pipe 21 instead of outside the container 15a.

The haemodialysis machine 1 has the particular advantage of having a low number of components to be sterilised and tested, substantially reducing the machine's downtime. The mixing and filtering assembly 15, of the use-and-throw type, does not actually have to be sterilised and tested before starting another dialysis treatment, these operations being carried out by the manufacturer.

Another advantage of the haemodialysis machine 1 described above and illustrated is that it permits a real-time check that the mixing and filtering assembly 15 is working correctly, actually eliminating the risk that the patient might receive reinfusion liquid that has not been correctly filtered.

## Claims

1. Haemodialysis machine (1) comprising a purification unit (2) through which is conveyed the blood of the patient that is to be purified of toxic substances in solution, a blood supply circuit (6, 7) capable of conveying the blood of the patient through said purification unit (2), a liquid reintegrating device (5) capable of mixing into the blood conveyed through said purification unit (2) a reinfusion liquid that is capable of restoring the correct percentage of salts and liquids in the blood, a first pipe for supplying a dialysis liquid to said purification unit (2), and a first pump (10) for conveying the dialysis liquid along said first pipe; the liquid reintegrating device (5) comprising a source (9) of reinfusion liquid, mixing means (15) arranged along said blood supply circuit (6, 7) and connected to said source (9) in order to mix the reinfusion liquid with the blood conveyed through the purification unit (2), and at least one filtering member (15) interposed between said source (9) and said mixing means (15); said filtering member (15) being incorporated into said mixing means (15), so that the filtering of the reinfusion liquid and the mixing of the reinfusion liquid itself with the blood conveyed through said purification unit (2) take place simultaneously; said liquid reintegrating device (5) further comprising a second pipe for supplying the reinfusion liquid from said source (9) to said mixing means (15); said haemodialysis machine being **characterised in that** said liquid reintegrating device (5) further comprises a second pump (16) for conveying the reinfusion liquid along said second pipe; said first and second pipe being arranged in parallel.

2. Haemodialysis machine according to Claim 1, **characterised in that** said mixing means (15), incorporating said filtering member (15), are arranged downstream of said purification unit (2).

3. Haemodialysis machine according to Claim 1, **characterised in that** said mixing means (15), incorporating said filtering member (15), are arranged upstream of said purification unit (2).

4. Haemodialysis machine according to any one of the foregoing Claims, **characterised in that** said mixing means (15), incorporating said filtering member (15), comprise a liquid-tight container (15a) connected to said source (9), and a bundle of capillary tubes (15b) that is positioned inside said liquid-tight container (15a), and capable of having pass through it the blood of the patient, which blood is conveyed through said purification unit (2); said capillary tubes (15b) being made of semipermeable material capable of permitting the passage of the reinfusion liquid inside said capillary tubes (15b), keeping outside any impurities in suspension.

5. Haemodialysis machine according to Claims 4, **characterised in that** said mixing means (15), incorporating said filtering member (15), comprise a recirculation circuit (20) capable of producing a flow of reinfusion liquid inside the liquid-tight container (15a), which flow is capable of preventing the deposition of impurities in suspension on the walls of the capillary tubes (15a).

6. Haemodialysis machine according to Claim 5, **characterised in that** said second pump (16) comprises a peristaltic pump (16).

7. Haemodialysis machine according to Claim 6, **characterised in that** said recirculation circuit (20) comprises a circulation pipe (21) connecting together two ends of said liquid-tight container (15a); said peristaltic pump (16) being capable of acting on said circulation pipe in order to supply, in parallel, the reinfusion liquid from said source (9) to said liquid-tight container (15a), and the reinfusion liquid along the circulation pipe (21).

8. Haemodialysis machine according to Claim 7, **characterised in that** it is provided with means (18) for sensing the presence of the blood of the patient in the reinfusion liquid contained inside said liquid-tight container (15a) in order to sense any fissuring of said capillary tubes (15b).

9. Haemodialysis machine according to Claim 8, **characterised in that** the liquid-tight container (15a) of said mixing means (15), incorporating said filtering member (15), is made at least partly of transparent material, and said means (18) for sensing the presence of blood comprise an optical sensor (18), which faces the transparent portion of said liquid-tight container (15a), and is capable of sensing a variation in colour of the reinfusion liquid contained inside the liquid-tight container (15a), caused by the presence of blood in solution.

10. Haemodialysis machine according to Claim 8, **characterised in that** said circulation pipe (21) is made at least partly of transparent material, and said means (18) for sensing the presence of blood comprise an optical sensor (18), which faces the transparent portion of said circulation pipe (21), and is capable of sensing a variation in colour of the reinfusion liquid circulating inside the circulation pipe (21), caused by the presence of blood in solution.

11. Haemodialysis machine according to any one of Claims 7 to 10, **characterised in that** said recirculation circuit (20) comprises a bleed valve (22) arranged along said circulation pipe (21); said bleed valve (22) being capable of connecting the pipe (21) with the outside, when commanded, so as to discharge the reinfusion liquid from said liquid-tight container (15a).

12. Haemodialysis machine according to any one of the preceding claims, **characterised in that** said purification unit (2) and said mixing means (15), incorporating said filtering member (15), constitute a single unit.

13. Haemodialysis machine according to any one of the preceding claims, **characterised in that** said purification unit (2) and said mixing means (15), incorporating said filtering member (15), are of the use-and-throw type.

## Patentansprüche

1. Hämodialysemaschine (1), umfassend eine Aufreinigungseinheit (2), durch welche das Blut eines Patienten geführt wird, das von gelösten toxischen Substanzen befreit werden soll, einen Blutversorgungskreislauf (6, 7), der in Lage ist, das Blut des Patienten durch die Aufreinigungseinheit (2) zu führen, eine Flüssigkeitsreintegrationsvorrichtung (5), die in der Lage ist, in das Blut, das durch die Aufreinigungseinheit (2) geführt wird, eine Reinfusionsflüssigkeit zu mischen, welche die korrekten Prozentsätze von Salzen und Flüssigkeiten in dem Blut wiederherstellen kann, eine erste Rohrleitung (pipe) zum Versorgen der Aufreinigungseinheit (2) mit einer Dialyseflüssigkeit und eine erste Pumpe (10) zum Führen der Dialyseflüssigkeit entlang der ersten Rohrleitung; wobei die Flüssigkeitsreingrationsvorrichtung (5) eine Quelle (9) der Reinfusionsflüssigkeit, ein Mischmittel (15), angeordnet entlang des Blutversorgungskreislaufes (6, 7) und verbunden mit dieser Quelle (9), um die Reinfusionsflüssigkeit mit dem Blut, das durch die Aufreinigungseinheit (2) geführt wird, zu mischen, und wenigstens eine Filtereinrichtung (15), die zwischen der Quelle (9) und der Mischvorrichtung (15) angeordnet ist, umfaßt; wobei die Filtereinrichtung (15) in das Mischmittel (15) mit einbezogen ist, so daß das Filtern der Reinfusionsflüssigkeit und das Mischen der Reinfusionsflüssigkeit selbst mit dem Blut, das durch die Aufreinigungseinheit (2) geführt wird, simultan stattfinden kann; wobei die Flüssigkeitsreintegrationsvorrichtung (5) weiterhin eine zweite Rohrleitung zum Versorgen des Mischmittels (15) mit der Reinfusionsflüssigkeit aus der Quelle (9) umfaßt; wobei die Hämodialysemaschine **dadurch gekennzeichnet ist, daß** die Flüssigkeitsreintegrationsvorrichtung (5) weiterhin eine zweite Pumpe (16) zum Führen der Reinfusionsflüssigkeit entlang der zweiten Rohrleitung umfaßt; wobei die erste und zweite Rohrleitung parallel zueinander angeordnet sind.

2. Hämodialysemaschine gemäß Anspruch 1, **dadurch gekennzeichnet, daß** das Mischmittel (15), das die Filtereinrichtung (15) beinhaltet, flußabwärts von der Aufreinigungseinheit (2) angeordnet ist.

3. Hämodialysemaschine gemäß Anspruch 1, **dadurch gekennzeichnet, daß** das Mischmittel (15), das die Filtereinrichtung (15) beinhaltet, flußaufwärts von der Aufreinigungseinheit (2) angeordnet ist.

4. Hämodialysemaschine gemäß einem der vorangehenden Ansprüche, **dadurch gekennzeichnet, daß** das Mischmittel (15), das die Filtereinrichtung (15) beinhaltet, eine flüssigkeitsdichte Aufnahmevorrichtung (15a) umfaßt, die mit der Quelle (9) verbunden ist, und ein Bündel Kapillarröhrchen (15b), das innerhalb der flüssigkeitsdichten Aufnahmevorrichtung (15a) angeordnet ist, und in der Lage ist, durch sich das Blut des Patienten laufen zu lassen, welches Blut durch die Aufreinigungseinheit (2) geführt wird; wobei die Kapillarröhrchen (15b) aus semipermeablem Material gemacht sind, das in der Lage ist, das Durchtreten der Reinfusionsflüssigkeit in die Kapillarröhrchen (15b) zu erlauben und jegliche Unreinheiten in der Suspension außen vor zu lassen.

5. Hämodialysemaschine gemäß Anspruch 4, **dadurch gekennzeichnet, daß** das Mischmittel (15), das die Filtereinrichtung (15) beinhaltet, einen Rezirkulationskreislauf (20) umfaßt, der in der Lage ist, einen Reinfusionsflüssigkeitsfluß innerhalb der flüssigkeitsdichten Aufnahmevorrichtung (15a) zu produzieren, wobei der Fluß in der Lage ist, die Ablagerung von Unreinheiten in der Suspension auf den Wänden der Kapillarröhrchen (15a) zu verhindern.

6. Hämodialysemaschine gemäß Anspruch 5, **dadurch gekennzeichnet, daß** die zweite Pumpe (16) eine peristaltische Pumpe (16) umfaßt.

7. Hämodialysemaschine gemäß Anspruch 6, **dadurch gekennzeichnet, daß** der Rezirkulationskreislauf (20) eine Zirkulationsrohrleitung (21) umfaßt, die zwei Enden der flüssigkeitsdichten Aufnahmevorrichtung (15a) miteinander verbindet; wobei die peristaltische Pumpe (16) in der Lage ist, auf die Zirkulationsrohrleitung einzuwirken, um so parallel die flüssigkeitsdichte Aufnahmevorrichtung (15a) mit der Reinfusionsflüssigkeit aus der Quelle (9) zu versorgen und die Reinfusionsflüssigkeit entlang der Zirkulationsrohrleitung (21) zu liefern.

8. Hämodialysemaschine gemäß Anspruch 7, **dadurch gekennzeichnet, daß** sie mit Mitteln (18) zum Detektieren der Gegenwart von Blut des Patienten in der Reinfusionsflüssigkeit ausgestattet ist, die innerhalb des flüssigkeitsdichten Aufnahmevorrichtung (15a) enthalten ist, um jegliche Rißbildung in den Kapillarröhrchen (15b) zu detektieren.

9. Hämodialysemaschine gemäß Anspruch 8, **dadurch gekennzeichnet, daß** die flüssigkeitsdichte Aufnahmevorrichtung (15a) des Mischmittels (15), das die Filtereinrichtung (15) beinhaltet, wenigstens teilweise aus transparentem Material gemacht ist und das Mittel (18) zum Fühlen der Gegenwart von Blut einen optischen Sensor (18) umfaßt, welcher dem transparenten Teil der flüssigkeitsdichten Aufnahmevorrichtung (15a) zugewandt ist und in der Lage ist, eine Farbveränderung der Reinfusionsflüssigkeit zu detektieren, die innerhalb der flüssigkeitsdichten Aufnahmevorrichtung (15a) angeordnet ist, verursacht durch die Gegenwart von Blut in der Lösung.

10. Hämodialysemaschine gemäß Anspruch 8, **dadurch gekennzeichnet, daß** die Zirkulationsrohrleitung (21) wenigstens teilweise aus transparentem Material gemacht ist und das Mittel (18) zum Fühlen der Gegenwart von Blut einen optischen Sensor (18) umfaßt, welcher dem transparenten Teil der Zirkulationsrohrleitung (21) zugewandt ist und in der Lage ist, eine Farbveränderung der Reinfusionsflüssigkeit zu detektieren, die innerhalb der Zirkulationsrohrleitung (21) zirkuliert, verursacht durch die Gegenwart von Blut in der Lösung.

11. Hämodialysemaschine gemäß einem der Ansprüche 7 bis 10, **dadurch gekennzeichnet, daß** der Rezirkulationskreislauf (20) ein Ablaßventil (22) umfaßt,. angeordnet entlang der Zirkulationsrohrleitung (21); wobei das Ablaßventil (22) in der Lage ist, die Rohrleitung (21) mit der Außenwelt bei Bedarf in Verbindung zu bringen, um so die Reinfusionsflüssigkeit aus der flüssigkeitsdichten Aufnahmevorrichtung (15a) zu entlassen.

12. Hämodialysemaschine gemäß einem der vorangehenden Ansprüche, **dadurch gekennzeichnet, daß** die Aufreinigungseinheit (2) und das Mischmittel (15), das die Filtereinrichtung (15) beinhaltet, eine einzelne Einheit bilden.

13. Hämodialysemaschine gemäß einem der vorangehenden Ansprüche, **dadurch gekennzeichnet, daß** die Aufreinigungseinheit (2) und das Mischmittel (15), das die Filtereinrichtung (15) beinhaltet, Einwegartikel sind.

## Revendications

1. Machine d'hémodialyse (1) comprenant une unité de purification (2) à travers laquelle est acheminé le sang du patient devant être purifié de substances toxiques en solution, un circuit d'approvisionnement en sang (6, 7) capable d'acheminer le sang du patient à travers ladite unité de purification (2), un dispositif de réintégration de liquide (5) capable de mélanger dans le sang acheminé à travers ladite unité de purification (2) un liquide de réinjection qui est capable de rétablir le pourcentage correct de sels et de liquides dans le sang, un premier tuyau pour alimenter ladite unité de purification (2) en liquide de dialyse, et une première pompe (10) pour acheminer le liquide de dialyse le long dudit premier tuyau ; le dispositif de réintégration de liquide (5) comprenant une source (9) de liquide de réinjection, des moyens de mélange (15) agencés le long dudit circuit d'approvisionnement en sang (6, 7) et reliés à ladite . source (9) afin de mélanger le liquide de réinjection avec le sang acheminé à travers l'unité de purification (2), et au moins un élément filtrant (15) intercalé entre ladite source (9) et lesdits moyens de mélange (15) ; ledit élément filtrant (15) étant incorporé auxdits moyens de mélange (15), de telle sorte que la filtration du liquide de réinjection et le mélange du liquide de réinjection proprement dit avec le sang acheminé à travers ladite unité de purification (2) aient lieu simultanément ;
ledit dispositif de réintégration de liquide (5) comprenant en outre un second tuyau pour alimenter lesdits moyens de mélange (15) en liquide de réinjection provenant de ladite source (9) ; ladite machine d'hémodialyse étant **caractérisée par le fait que** ledit dispositif de réintégration de liquide (5) comprend en outre une seconde pompe (16) pour acheminer le liquide de réinjection le long dudit second tuyau ; lesdits premier et second tuyaux étant parallèles.

2. Machine d'hémodialyse selon la revendication 1, **caractérisée par le fait que** lesdits moyens de mélange (15), incorporant ledit élément filtrant (15), sont disposés en aval de ladite unité de purification (2).

3. Machine d'hémodialyse selon la revendication 1, **caractérisée par** le fiat que lesdits moyens de mélange (15), incorporant ledit élément filtrant (15), sont disposés en amont de ladite unité de purification (2).

4. Machine d'hémodialyse selon l'une quelconque des revendications précédentes, **caractérisée par le fait que** lesdits moyens de mélange (15), incorporant ledit élément filtrant (15), comprennent un conteneur étanche aux liquides (15a) relié à ladite source (9), et un faisceau de tubes capillaires (15b) qui sont placés à l' intérieur dudit conteneur étanche aux liquides (15a) et à travers lesquels le sang du patient peut passer, sang qui est acheminé à travers ladite unité de purification (2) ; lesdits tubes capillaires (15b) étant fabriqués à partir d'un matériau semi-perméable capable de permettre le passage du liquide de réinjection à l'intérieur desdits tubes capillaires (15b), laissant à l'extérieur toute impureté en suspension.

5. Machine d'hémodialyse selon la revendication 4, **caractérisée par le fait que** lesdits moyens de mélange (15), incorporant ledit élément filtrant (15), comprennent un circuit de recirculation (20) capable de produire un débit de liquide de réinjection à l'intérieur du conteneur étanche aux liquides (15a), lequel débit est capable d'empêcher le dépôt d'impuretés en suspension sur les parois des tubes capillaires (15a).

6. Machine d'hémodialyse selon la revendication 5, **caractérisée par le fait que** ladite seconde pompe (16) comprend une pompe péristaltique (16).

7. Machine d'hémodialyse selon la revendication 6, **caractérisée par le fait que** ledit circuit de recirculation (20) comprend un tuyau de circulation (21) reliant ensemble deux extrémités dudit conteneur étanche aux liquides (15a) ; ladite pompe péristaltique (16) étant capable d'agir sur ledit tuyau de circulation afin de fournir, en parallèle, audit conteneur étanche aux liquides (15a) le liquide de réinjection provenant de ladite source (9), et le liquide de réinjection le long du tuyau de circulation (21).

8. Machine d'hémodialyse selon la revendication 7, **caractérisée par le fait qu'**elle est munie de moyens (18) destinés à détecter la présence du sang du patient dans le liquide de réinjection contenu à l'intérieur dudit conteneur étanche aux liquides (15a) afin de détecter toute fissuration desdits tubes capillaires (15b).

9. Machine d'hémodialyse selon la revendication 8, **caractérisée par le fait que** le conteneur étanche aux liquides (15a) desdits moyens de mélange (15), incorporant ledit élément filtrant (15), est fabriqué au moins en partie à partir d'un matériau transparent, et lesdits moyens (18) destinés à détecter la présence de sang comprennent un capteur optique (18), situé en face de la partie transparente dudit conteneur étanche aux liquides (15a), et capable de détecter une modification de la couleur du liquide de réinjection contenu à l'intérieur du conteneur étanche aux liquides (15a), provoqués par la présence de sang dans la solution.

10. Machiné d'hémodialyse selon la revendication 8, **caractérisée par le fait que** ledit tuyau de circulation (21) est fabriqué au moins en partie à partir d'un matériau transparent, et lesdits moyens (18) destinés à détecter la présence de sang comprennent un capteur optique (18), situé en face de la partie transparente dudit tuyau de circulation (21) et capable de détecter une modification de la couleur du liquide de réinjection circulant à l'intérieur du tuyau de circulation (21), provoquée par la présence de sang dans la solution.

11. Machine d'hémodialyse selon l'une quelconque des revendications 7 à 10, **caractérisée par le fait que** ledit circuit de recirculation (20) comprend une soupape de décharge (22) disposée le long dudit tuyau de circulation (21) ; ladite soupape de décharge (22) étant capable de relier le tuyau (21) avec l'extérieur, lorsqu'elle est activée, de manière à évacuer le liquide de réinjection dudit conteneur étanche aux liquides (15a).

12. Machine d'hémodialyse selon l'une quelconque des revendications précédentes, **caractérisée par le fait que** ladite unité de purification (2) et lesdits moyens de mélange (15) incorporant ledit élément filtrant (15) forment une seule unité.

13. Machine d'hémodialyse selon l'une quelconque des revendications précédentes, **caractérisée par le fait que** ladite unité de purification (2) et lesdits moyens de mélange (15) incorporant ledit élément filtrant (15) sont du type jetable après utilisation.
